# EUROPÄISCHE PATENTSCHRIFT

(11) **EP 1 015 640 B1**
(45) Veröffentlichungstag und Bekanntmachung des Hinweises auf die Patenterteilung: **04.08.2010**
(21) Anmeldenummer: 98951443.5
(22) Anmeldetag: 21.09.1998
(51) Int. Cl.: C12Q 1/68, C12P 19/34

(54) **VERFAHREN ZUR SYNTHESE UND AMPLIFIKATION VON NUKLEINSÄUREN**
METHOD FOR SYNTHESISING AND AMPLIFYING NUCLEIC ACIDS
PROCEDE DE SYNTHESE ET D'AMPLIFICATION D'ACIDES NUCLEIQUES

(30) Priorität: 22.09.1997 DE 19741714
(43) Veröffentlichungstag der Anmeldung: 05.07.2000
(73) Patentinhaber: CLONDIAG GmbH, 07749 Jena (DE)
(72) Erfinder: ELLINGER, Thomas, D-07743 Jena (DE); EHRICHT, Ralf, D-09306 Rochlitz (DE)
(74) Vertreter: Maiwald, Walter
(86) Internationale Anmeldenummer: PCT/EP1998/006006
(87) Internationale Veröffentlichungsnummer: WO 1999/015698

(56) Entgegenhaltungen:
- WO-A-96/17087
- WO-A-97/24455
- FR-A- 2 678 639
- FAHY E ET AL: "SELF-SUSTAINED SEQUENCE REPLICATION (3SR): AN ISOTHERMAL TRANSCRIPTION-BASED AMPLIFICATION SYSTEM ALTERNATIVE TO PCR" PCR METHODS AND APPLICATIONS, Bd. 1, Nr. 1, 1. Januar 1991, Seiten 25-33, XP000600266 in der Anmeldung erwähnt
- EHRICHT R ET AL.: "Cooperative amplification of templates by cross-hybridization (CATCH)" EUROPEAN JOURNAL OF BIOCHEMISTRY, Bd. 243, 1997, Seiten 358-364, XP002098795

## Beschreibung

Die vorliegende Erfindung betrifft ein Verfahren zur Synthese und Amplifikation von Nukleinsäuren mittels einer Enzym-katalysierten Reaktion, bei der eine Nukleinsäure ausgehend von einer einzelsträngigen Initiatornukleinsäure kopiert wird, ohne daß eine Hybridisierung der Initiatornukleinsäure an die zu kopierende Nukleinsäure stattfindet. Die Erfindung betrifft somit ein Verfahren, bei dem eine lineare Nukleinsäure ausgehend von einer zumindest am 3'-Ende einen einzelsträngigen Bereich aufweisenden Initiatornukleinsäure durch eine Polymerase-Aktivität kopiert wird, wobei die Initiatornukleinsäure keine Homologie mit der zu kopierenden Nukleinsäure aufweisen muß. Die diesem Verfahren zugrundeliegende Reaktion wird im folgenden als Initiator-gesteuerte Amplifikation (initiator-directed amplification, IDA) bezeichnet.

Die Prozesse der DNA-Replikation, DNA-Ligation und RNA-Transkription und das zunehmende Wissen um die diesen Prozessen zugrundeliegenden Reaktionsmechanismen und - prinzipien haben die Basis für verschiedene in vitro-Nukleinsäureamplifikationsstrategien geliefert.

Die zuerst realisierte Methode, allgemein als Polymerasekettenreaktion (polymerase-chain-reaction, PCR) bekannt, basiert auf einer DNA-Polymerase-katalysierten Replikation beider Stränge einer Target-DNA-Sequenz, die durch zwei mit der Target-DNA-Sequenz homologe Oligonukleotidprimer initiiert wird. Dabei wird die Reaktionstemperatur zyklisch variiert, um die Denaturierung des DNA-Templates, gefolgt durch die Hybridisierung der Primer an die Targetsequenz und schließlich die Polymerase-katalysierte DNA-Synthese zu erlauben. Diese in vitro-Amplifikationstechnik verlangt somit neben einem speziellen, zyklischen Temperaturregime die Hybridisierung spezifischer Oligonukleotidprimer an eine spezifische DNA-Templatesequenz. Die in vitro-Amplifikation mittels PCR sowie Modifikationen und Weiterentwicklungen dieser Reaktion sind vielfach beschrieben worden, beispielsweise in Saiki, R.K. et al. (1985) Science 230, 1350-1354; Mullis, K.B. and Faloona, A. (1987) Methods Enzymol. 155, 335-350; EP-A2-0 200 362.

Eine andere Amplifikationsmethode, genannt Ligasekettenreaktion (ligase-chain-reaction, LCR), beruht wie die oben erwähnte PCR auf einem thermozyklischen Prozeß, wobei im Fall der LCR Ligationsprodukte von zwei Paaren von komplementären Oligonukleotiden akkumulieren. Wie bei der PCR müssen auch hier Oligonukleotidprimer eingesetzt werden, deren Sequenz mit der Sequenz der Templatenukleinsäure identisch oder zumindest im wesentlichen identisch ist, so daß eine spezifische Hybridisierung der Primer an die zu amplifizierenden Sequenzabschnitte stattfinden kann. Die LCR-Methode, mittels derer ausschließlich die durch die hinzugefügten Oligonukleotide repräsentierten Sequenzen amplifiziert werden können, ist ausführlich beschrieben worden, inter alia, in Barany, F. (1991) PCR Methods Appl. 1, 5-16; EP-A2-0 439 182.

Aus WO 90/01069 ist darüber hinaus eine Amplifikationsmethode (P&LCR) bekannt, die die Merkmale der PCR und der LCR in sich vereinigt, also auf Polymerase- und auf Ligase-katalysierten Reaktionen beruht.

In WO97/24455 wird ein Verfahren zur Synthese einer Nukleinsäure beschrieben bei der ein Oligonukleotid verwendet wird welches mit der 5' CAP Struktur eines RNA Moleküls hybridisiert und von einer reversen Transkriptase als Verlängerung des Templates erkannt und verwendet wird.

FR-A-2678639 (Rhone Poulenc Rorer) beschreibt die Ligation eines Oligonukleotids an das 3' Ende einer Zielnukleinsäure, wobei die Sequenz des Oligonukleotids in einem weiteren Schritt als Primerbindungsstelle fungiert.

Während es sich bei PCR, LCR und P&LCR um thermozyklische Amplifikationsmethoden handelt, gelang es mittlerweile, auch isothermale Strategien in die Praxis umzusetzen. Hier sind insbesondere die "self-sustained sequence replication" (3SR), beschrieben u.a. in Guatelli, J.C. et al. (1990) Proc. Natl. Acad. Sci. USA 87, 1874-1878; Fahy, E. et al. (1991) PCR Methods Appl. 1, 25-33; EP-A2-0 373 960 und WO 92/08800, und die "nucleid acid sequence-based amplification" (NASBA), beschrieben u.a. in Compton, J. (1991) Nature 350, 91-92, zu erwähnen.

Sowohl die 3SR- als auch die NASBA-Strategie basieren auf in Retroviren beobachteten Replikationsprinzipien. In beiden Fällen initiiert die Hybridisierung eines zu dem 3'-Ende einer Target-RNA-Sequenz komplementären Primers eine Reverse Transkriptase-katalysierte DNA-Synthese (first strand cDNA synthesis). Im nachfolgenden Schritt wird das RNA-Template durch die Aktivität der RNase H aus *Escherichia coli* abgebaut, wodurch einem zweiten Primer, der an seinem 5'-Ende die Erkennungssequenz für eine virale RNA-Polymerase, beispielsweise T7-RNA-Polymerase, umfaßt, die Hybridisierung an das neusynthetisierte cDNA-Molekül ermöglicht wird. Nachdem der DNA-Doppelstrang durch die Aktivität der Reverse Transkriptase (second strand cDNA synthesis) vervollständigt worden ist und auch die T7-Promotorregion doppelsträngig vorliegt, synthetisiert die RNA-Polymerase zahlreiche RNA-Moleküle, deren Sequenz der der ursprünglichen Target-RNA entspricht und die wiederum als Template in nachfolgenden Amplifikationszyklen dienen. Auf diese Weise resultiert der spontane Wechsel zwischen Reverse Transkriptase-, RNase H- und RNA-Polymerase-katalysierten Reaktionen bei einer festgesetzten Temperatur in einer exponentiellen Amplifikation der Targetsequenz.

Eine weitere isothermale Amplifikationsmethode ist die als "strand displacement amplification" (SDA) bezeichnete Technik von Walker, G.T. et al. (1992) Nucl. Acids Res. 20, 1691-1696 und Proc. Natl. Acad. Sci. USA 89, 392-396; vgl. auch EP-A1-0 497 272 und EP-A2-0 500 224. In dieser Strategie wird das den isothermalen Amplifikationsmethoden anhaftende Problem der Strangdissoziation der gebildeten Nukleinsäuredupleces durch die Verdrängung ("Displacement") des komplementären DNA-Stranges von der Targetsequenz während der Synthese eines neuen Stranges gelöst. Auch im Falle der SDA-Methode hybridisiert ein mit der Targetsequenz komplementärer Oligonukleotidprimer an einen Strang der zu Beginn denaturierten Target-DNA. Anschließend verlängert eine DNA-Polymerase, vorzugsweise das große Fragment der DNA-Polymerase I aus *E. coli* mit 5'-Exonukleasedefizienz (Exo- Klenow), sowohl den Primer als auch das Templatemolekül durch Kopieren des 5'-Endes des Primers, der an seinem 5'-Ende eine bestimmte Restriktionsschnittstelle umfaßt. Da während der DNA-Synthese bestimmte modifizierte dATP-Nukleotide angeboten und eingebaut werden, ist nach Zugabe der speziellen Restriktionsendonuklease nur die im Primer enthaltene Schnittstelle für einen Restriktionsverdau, nicht aber die DNA-Kopie für eine Restriktionsverdau zugänglich (sog. Hemirestriktion). An diesem "nick" wird durch die DNA-Polymerase, die nicht zur Nicktranslation befähigt ist, die DNA-Synthese initiiert und der nicht als Template dienende Strang durch Strangdisplacement während der DNA-Synthese abgelöst.

Aus WO 90/10064, 91/03573 und 91/16446, vgl. auch Blanco et al. (1994) Proc. Natl. Acad. Sci. USA 91, 12198-12202, ist eine weitere isothermale DNA-Amplifikationsmethode bekannt, bei der DNA-Replikationsproteine des Bakteriophagen φ29 eingesetzt werden und mit deren Hilfe auch sehr lange DNA-Segmente (> 70 kb) amplifiziert werden können. Dieses Verfahren erfordert u.a. die Anwesenheit eines sog. terminalen Proteins (TP) von φ29, das als Primer wirkt, einer DNA-Polymerase von φ29 und weiterer Replikationsproteine, wie SSB (single-stranded DNA-binding protein) und DBP (double-stranded DNA-binding protein). Bei dieser Amplifikationsmethode findet im Unterschied zu den oben beschriebenen Verfahren eine Hybridisierung spezifischer Oligonukleotide an die zu kopierende Nukleinsäure nicht statt, als Primer dient stattdessen das terminale Protein (TP) aus φ29.

Aus Kramer, F.R. und Lizarde, P.M. (1989) Nature 339, 401-402 und Lomeli, H. (1989) Clin. Chem. 35, 1826-1831 ist des weiteren eine isothermale RNA-Amplifikationsmethode bekannt, bei der eine RNA-abhängige RNA-Polymerase aus dem Bakteriophagen Q_{β} (Q_{β}-Replikase) eingesetzt wird. Die in diesem Verfahren verwendeten RNA-Sonden enthalten die für die Replikation durch Q_{β}-Replikase erforderlichen Sequenzelemente sowie ein internes Segment, das zu der zu detektierenden Targetsequenz komplementär ist. Unter bestimmten Reaktionsbedingungen werden durch die Aktivität der Q_{β}-Replikase nur solche Moleküle kopiert, die mit der entsprechenden Targetsequenz in der zugegebenen Probe hybridisieren. Die erzeugten Kopien dienen anschließend in nachfolgenden Syntheseschritten zusammen mit dem Ausgangsmolekül als Template für weitere Replikationszyklen. Auf diese Weise kann eine 10⁸-fache Amplifikation der Sondenmoleküle während 15 Minuten Inkubationen bei einer festgesetzten Temperatur erreicht werden.

Sämtliche bekannten Amplifikationstechniken erfordern entweder die Hybridisierung eines als Sonde dienenden Primermoleküls an die Targetsequenz oder den Einsatz spezieller Replikationsproteine aus Bakteriophagen (protein-primed amplification). Es wäre äußerst wünschenswert, über ein Amplifikationsverfahren verfügen zu können, das ohne die Hybridisierung spezifischer Oligonukleotidprimer an das zu amplifizierende Nukleinsäuremoleküle auskommt und auch die oben beschriebene, komplexe Replikationsmechanerie aus Bakteriophagen nicht erfordert. Ein solches Verfahren, das somit theoretisch die Synthese und Amplifikation jeder beliebigen Nukleinsäure ermöglichen würde, wäre für zahlreiche Anwendungen, beispielsweise auf den Gebieten der molekularen Biotechnologie, inbesondere der gentechnischen in vitro-Behandlung von Nukleinsäuren, der evolutiven Biotechnologie, der kombinatorischen Chemie und der molekularen Informationsverarbeitung von unschätzbarem Wert. Ein solches Verfahren könnte auch in Kombination mit bekannten primerabhängigen Amplifikationsmethoden angewandt werden, insbesondere wenn spezifische Sequenzen amplifiziert werden sollen.

Aufgabe der vorliegenden Erfindung ist es, ein Verfahren bereitzustellen, das die Synthese und gegebenfalls Amplifikation von Nukleinsäuren ermöglicht, ohne daß hierzu eine Hybridisierung komplementärer Nukleinsäuremolelüle an die zu kopierende Nukleinsäure stattfinden muß.

Eine weitere Aufgabe der Erfindung besteht darin, Anwendungsmöglichkeiten für eine solche hybridisierungsunabhänige Synthese- bzw. Amplifikationstechnik aufzuzeigen.

Diese Aufgaben werden gemäß den unabhängigen Ansprüchen der vorliegenden Erfindung gelöst. Die Unteransprüche definieren vorteilhafte Ausführungsformen der Erfindung.

Experimentell wurde überraschend festgestellt, daß in einer Polymerase-katalysierten Reaktion die Stränge eines doppelsträngigen Templates ausgehend vom freien 3'-OH einer einzelsträngigen Nukleinsäure (Initiator) kopiert werden, wobei die Reaktion im Unterschied zu allen bisher bekannten Reaktionen dieser Art an doppelsträngigen DNA-Molekülen keine Hybridisierung der einzelsträngigen Nukleinsäure an das Template beinhaltet.

Ausgangspunkt für diese Beobachtung waren Versuche, in denen ein doppelsträngiges Template mittels der oben beschriebenen 3SR-Reaktion amplifiziert wurde. Die Reaktion enthielt neben dNTPs und NTPs, Puffer und Template zwei unphosphorylierte Oligonukleotidprimer mit einer Länge von je 20 Basenpaaren, eine Reverse Transkriptase und T7-RNA-Polymerase. Unter bestimmten Reaktionsbedingungen zeichneten sich die erhaltenen doppelsträngigen Reaktionsprodukte dadurch aus, daß sie in Schritten von 20 Basenpaaren verlängert waren. Die Reaktionsprodukte wurden kloniert und einer Sequenzanalyse unterzogen. Diese Sequenzanalyse zeigte, daß diese Moleküle neben der erwarteten Templatesequenz an deren Enden mehrere Kopien der beiden Primersequenzen als direkte Wiederholungen enthielten. Template- und Primersequenz sowie die Primersequenzen untereinander waren größtenteils unmittelbar, d.h. ohne Überlappung, miteinander verknüpft bzw. zeigten eine Deletion bzw. Insertion von einzelnen Basen an der Verknüpfungsstelle. Nachfolgend durchgeführte Amplifikationsexperimente, bei denen unter gleichen Bedingungen nur die Oligonukleotidprimer, jedoch kein Template zugegeben wurden, führten zur Bildung von repetitiven DNA-Molekülen, deren Länge in Schritten von 20 Basenpaaren variierte. Sie enthielten ein zentralen Element von 33 Basen Länge, das durch Hybridisierung von zwei Kopien eines der beiden Primer über die 3'-Enden und nachfolgendes Auffüllen zum Doppelstrang entstand, sowie eine Vielzahl direkt miteinander fusionierter Sequenzen der beiden Primer.

Die in diesen und weiteren Experimenten gewonnenen Daten, die in den nachfolgenden Ausführungsbeispielen ergänzend erläutert werden, lassen auf einen Reaktionsmechanismus schließen, bei dem die eingesetzte Polymerase in einer Template-abhängigen Reaktion sehr effektiv einzelsträngige Nukleinsäuremoleküle mit freiem 3'-OH verlängert, ohne daß die einzelsträngige Nukleinsäure mit dem Template hybridisiert. Die Sequenz des Einzelstranges muß somit keinerlei Homologie zur Templatesequenz besitzen. Der Einzelstrang wirkt daher nicht als typischer Primer, sondern kann besser als Initiator bezeichnet werden. Als Template dient lineare doppelsträngige DNA. Das Reaktionsprodukt enthält die Sequenz des Initiators, die direkt mit dem 5'-Ende des Templates fusioniert ist. Der Prozeß wird iterativ durchlaufen und resultiert in immer längeren Sequenzen und einer Netto-Amplifikation des Templates. Um den Unterschied zu hybridisierungsabhängigen Amplifikationsschemata zu verdeutlichen, kann die Reaktion somit als initiatorgesteuerte oder initiatorabhängige Amplifikation ("initiator directed amplification" oder "initiator dependent amplification", IDA) bezeichnet werden.

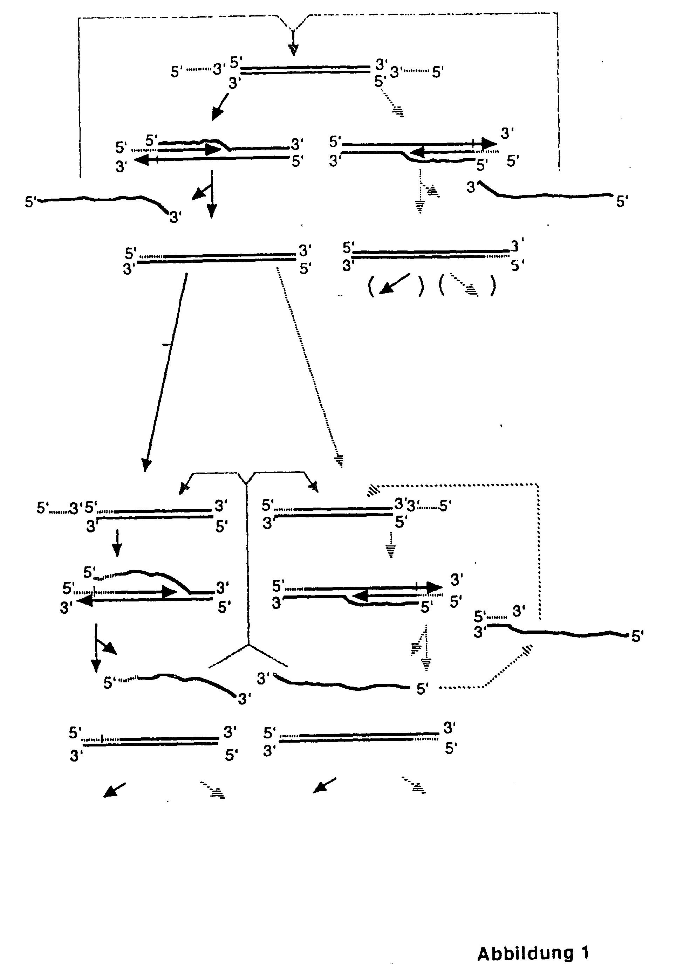

Ohne die Erfindung an eine theoretische Erklärung binden zu wollen, wird gegenwärtig der in Abbildung 1 veranschaulichte Reaktionsmechanismus angenommen. Das Schema zeigt den vermuteten Mechanismus der IDA-Reaktion für die ersten zwei Reaktionszyklen. Die Polymerase bindet gleichzeitig sowohl das Ende des doppelsträngigen Templates als auch das einzelsträngige Initiatormolekül (vertikal schraffiert). Das 3'-Ende des Templates wird verlängert, so daß der Initiator zum Doppelstrang aufgefüllt wird. Ausgehend vom 3'-Ende des Intiators erfolgt eine Synthese in den DNA-Doppelstrang hinein, die je nach Bindung von Polymerase und Initiator entweder "linksseitig" (schwarze Pfeile) oder "rechtsseitig" (horizontal schraffierte Pfeile) erfolgen kann. Ein Strang dient dabei als Template, der andere wird vermutlich als Einzelstrang abgelöst. Resultat der Reaktion ist ein DNA-Doppelstrang, bei dem die Sequenz des ursprünglichen Doppelstrangtemplates direkt mit der des Initiators fusioniert ist. Die im "linksseitigen" und "rechtsseitigen" Zyklus entstehenden Einzelstränge sind komplementär und können zum Doppelstrang hybridisieren (graue Pfeile). Im zweiten Zyklus kann ausgehend vom "linksseitig" oder "rechtsseitig" (Pfeile in Klammern, nicht gezeigt) verlängerten Template wiederum eine "linksseitige" (schwarze Pfeile) oder "rechtsseitige" (horizontal schraffierte Pfeile) Verlängerung erfolgen. Der Mechanismus ist ähnlich wie in dem ersten Zyklus, wobei ein Teil der Einzelstränge jedoch die zum Initiator komplementäre Sequenz enthält. Diese Einzelstränge können nach Bindung des Intiators als Primer durch die Polymerase zum Doppelstrang aufgefüllt werden (schräg schraffierte Pfeile).

Die Abbildung stellt ein idealisiertes und vereinfachtes Schema dar. In der Realität ist die Reaktion voraussichtlich wesentlich komplexer, da mehrere Zyklen an einem Molekül gleichzeitig ablaufen können und wesentlich mehr Wechselwirkungsmöglichkeiten zwischen den einzelsträngigen Intermediaten bestehen als in der Darstellung gezeigt. In jedem Fall verdeutlicht das in Abbildung 1 dargestellte Reaktionsschema die beobachtete Entstehung von repetitiven Sequenzen sowie die beobachtete Fusionierung von DNA-Sequenzen ohne Sequenzhomologie. Diese Beobachtungen können mit herkömmlichen Synthesemechanismen nicht erklärt werden; eine Polymerase-katalysierte template-abhängige Primerverlängerung ohne Hybridisierung zwischen Primer und Template wird durch die vorliegende Erfindung erstmals bereitgestellt.

Die erfindungsgemäße IDA-Reaktion ist isothermal und führt durch iterative Wiederholung zu einer Netto-Amplifikation. Eine vorteilhafte Eigenschaft dieser Reaktion besteht vor allem darin, daß sie von einer spezifischen Hybridisierung unabhängig ist, wodurch im Verlauf der Reaktion potentiell jede beliebige Sequenz mit jeder anderen fusioniert werden kann. Darüber hinaus ermöglicht die IDA-Reaktion die Synthese an doppelsträngigen Templatemolekülen. Durch diese einzigartige Kombination der Eigenschaften besitzt die Reaktion ein großes Anwendungspotential und eröffnet völlig neue Möglichkeiten für sämtliche Bereiche der gentechnischen in vitro-Behandlung von Nukleinsäuren, der evolutiven Biotechnologie sowie verwandter Aufgabenfelder.

So kann die erfindungsgemäße IDA-Reaktion allgemein zur Amplifikation von Nukleinsäuren eingesetzt werden. Der Vorteil der Reaktion gegenüber allen bekannten Amplifikationsmethoden besteht insbesondere in ihrer Unabhängigkeit von der Struktur der Enden des Templates. Dadurch entfällt die Notwendigkeit, für jedes spezifische Amplifikationsproblem spezifische Primersequenzen herstellen zu müssen. Ebenso können mit Hilfe der IDA-Reaktion Pools von Nukleinsäuren mit heterologen Enden in einer einzigen Reaktion amplifiziert werden.

Versuche haben bestätigt, daß die erfindungsgemäße Reaktion allein als Amplifikationsmethode dienen kann. Hierbei kann die schrittweise Verlängerung des Templates während der Reaktion durch Zusatz einer Restriktase, durch deren Aktivität das 5'-Ende des Templates regeneriert wird, verhindert werden. Dieser Effekt könnte auch durch den Einsatz von RNA-Initiatormolekülen in Kombination mit einem RNAabbauenden Enzym, beispielsweise RNAse, erreicht werden. In diesem Fall würde der RNA-Initiator nach Auffüllen zum Doppelstrang zumindest partiell durch eine RNAse abgebaut werden. Als Initiatornukleinsäure können auch Nukleinsäureanaloga, beispielsweise Peptide Nucleic Acid (PNA; Wittung et al. (1994) Nature 368, 561-563), eingesetzt werden.

Darüber hinaus kann die erfindungsgemäße Reaktion auch einer PCR oder einer anderen primerabhängigen Amplifikationsreaktion vorgeschaltet werden. Im ersten Schritt wird dadurch das jeweilige Template mit der Initiatorsequenz fusioniert. Anschließend erfolgt eine spezifische Amplifikation, bei der die Initiatoren als Primer eingesetzt werden können.

Aufgrund ihrer besonderen Eigenschaften kann die erfindungsgemäße Reaktion für zahlreiche Anwendungen eingesetzt werden, die durch bereits etablierte Amplifikationstechniken nicht abgedeckt werden. Beispielhaft seien hier die Amplifikation bzw. das Kopieren von Total-DNA oder Nukleinsäuregemischen, z.B. für diagnostische Zwecke oder im Zusammenhang mit Methoden der in vitro-Evolution (z.B. die primerunabhängige Amplifikation in SELEX-Prozessen ("systemic evolution of ligands by exponential enrichment"; vlg. z.B. WO 95/30775) oder andere in vitro-Selektionsverfahren) genannt. Es ergibt sich von selbst, daß die erfindungsgemäße Reaktion allgemein in jedem beliebigen Prozeß eingesetzt werden kann, bei dem die Vervielfältigung sämtlicher in einer Probe enthaltenen Nukleinsäuremoleküle oder allgemein eine zufällige Synthese und Amplifikation von Nukleinsäuremolekülen erwünscht ist.

Alternativ zu oder gemeinsam mit doppelsträngigen Nukleinsäuren können ebenso einzelsträngige Nukleinsäuren als Ausgangstemplate dienen. In diesem Fall kann z.B. eine Initiatornukleinsäure eingesetzt werden, die zu dem 3'-Ende des zu kopierenden Einzelstranges komplementär ist, so daß durch Hybridisierung der Initiatornukleinsäure an das Templatemolekül und anschließende Polymerase-katalysierte Auffüllung des Doppelstranges ein doppelsträngiges Template entsteht, das anschließend nach dem erfindungsgemäßen IDA-Mechanismus amplifiziert wird. Dieses Verfahren könnte zur Umschreibung von RNA in doppelsträngige DNA bei gleichzeitiger Amplifikation eingesetzt werden und würde somit eine Alternative zur RT-PCR (Reverse Transkriptase-PCR) darstellen.

Allgemein kann die erfindungsgemäße Reaktion darüber hinaus als Grundlage für alle Verfahren und Prozesse dienen, die bislang auf Kopiervorgängen basierten, die einen sequenzspezifischen Vorgang als Startreaktion benötigten. Solche Verfahren umfassen z.B. auch Sequenzierungs- und footprint-Methoden, die Herstellung von markierten Nukleinsäuren und die Herstellung einzelsträngiger DNA, die z.B. mit Selektionsverfahren (z.B. SELEX-Prozesse) gekoppelt werden kann.

Des weiteren kann die erfindungsgemäße IDA-Reaktion zur Fusionierung von nichtkomplementären Nukleinsäuren eingesetzt werden. Dadurch eröffnet sie eine Reihe neuer Möglichkeiten, insbesondere für die molekulare Biotechnologie. So gestattet das erfindungsgemäße Verfahren beispielsweise die Herstellung von Random-Sequenzen aus kurzen randomisierten Oligonukleotiden und damit die Erschließung von Sequenzräumen, die bisher praktisch nicht zugänglich waren.

Eine weitere Anwendungsmöglichkeit des erfindungsgemäßen Verfahrens besteht in der enzymatischen Template-unabhängigen Synthese von Oligonukleotiden. Diese Anwendung eröffnet ein weites Feld von Möglichkeiten in der kombinatorischen Chemie sowie für die molekulare Informationsverarbeitung (DNA-computing, DNA-chip-Technologie). Im Zusammenwirken mit Prozessen, die eine Rekombination von Nukleinsäuren zur Folge haben, können dabei Sequenzen beliebiger Sequenz und Länge erzeugt werden. Ebenso ermöglicht das erfindungsgemäße Verfahren die Herstellung langer doppelsträngiger DNA-Moleküle (beispielsweise synthetischer Gene) aus Einzelsträngen ohne Sequenzkomplementarität.

Weitere Anwendungsmöglichkeiten des erfindungsgemäßen Verfahrens liegen in der Herstellung von spezifischen proteinkodierenden Sequenzen durch gesteuerte oder randomisierte Fusion von verschiedenen Codon-Bausteinen. Das gesamte Spektrum proteinogener Aminosäuren kann mit 21 Bausteinen abgedeckt werden. So wäre beispielsweise eine Kopplung der erfindungsgemäßen Reaktion mit Translationsmethoden möglich, so daß gleichzeitig die für das Protein kodierende Sequenz, als auch das Protein selbst hergestellt werden. Solche Translationsmethoden umfassen sowohl das klassische Translationsverfahren (siehe beispielsweise Baranov et al. (1989) Gene 84, 463-466; Morozov et al. (1993) Proc. Natl. Acad. Sci. USA 90, 9325-9329), als auch Verfahren, die auf alternativen Mechanismen beruhen, wie beispielsweise die Ribozym-katalysierte Synthese von Proteinen, an deren Entwicklung gegenwärtig intensiv gearbeitet wird (siehe z.B. Illangasekare et al. (1995) Science 267, 643-647; Lohse und Szostak (1996) Nature 381, 442-444; Hager et al. (1996) Chem. Biol. 3, 717-725), wobei das Ribozym identisch mit der im jeweiligen Schritt fusionierten Nukleinsäure sein kann.

Besonders wertvoll ist die erfindungsgemäße IDA-Methode im Zusammenhang mit shuffling-Technologien (vgl. beispielsweise Stemmer (1994) Nature 370, 389-391; Stemmer (1994) Proc. Natl. Acad. Sci. USA 91, 10747-10757; WO-A1-95/22625). Die IDA-Methode gestattet das shuffling von DNA-Fragmenten ohne Sequenzhomologie. Ein dosierter Einsatz in Kombination mit sequenzspezifischem shuffling würde den globalen Umbau von Biomolekülen erlauben. Diese Technik ist sowohl für Proteine, als auch für funktionelle Nukleinsäuren und Nukleinsäureanaloga anwendbar. Dadurch wäre es einerseits möglich, multifunktionale Biomoleküle auf eine gewünschte Form zu optimieren und mit geeigneten Selektionsverfahren unerwünschte Funktionen zu beseitigen. Andererseits könnten verschiedene Biomoleküle mit unterschiedlicher Funktion und Struktur miteinander so fusioniert werden, daß ineinandergeschaltete multifunktionale Moleküle entstehen. Ergebnis dieses neuen Verfahrens können multifunktionale Proteine, katalytische Nukleinsäuren oder Aptamere sein.

Diese beispielhafte Auflistung möglicher Anwendungsgebiete des erfindungsgemäßen Verfahrens macht das große Potential der IDA-Methode deutlich. Umso bemerkenswerter ist die verhältnismäßig einfache Durchführung des Verfahrens und die geringe Anzahl der hierzu erforderlichen Komponenten.

Erfindungsgemäß läuft die IDA-Reaktion in einem Reaktionsansatz ab, der folgende Komponenten enthält: ein Enzym mit Polymerase-Aktivität, d.h. ein Enzym, das die Synthese von Nukleinsäuremolelülen aus kleineren Bausteinen katalysiert, die entsprechenden Bausteine bzw. Monomere, üblicherweise also dATP, dCTP, dGTP und dTTP (ggf. in modifizierter Form), mindestens eine am 3'-Ende einen einzelsträngigen Bereich aufweisende Initiatornukleinsäure und mindestens ein lineares Templatenukleinsäuremolekül. Dabei kann das lineare Template auch auf zwei Kopien eines Initiatormoleküls beruhen.

Entsprechend enthält ein typischer IDA-Reaktionsansatz folgende Komponenten:

| | |
|---|---|
| 40 | mM Tris pH 7,5 |
| 15 | mM MgCl₂ |
| 16,6 | mM NaCl |
| 1 | mM dNTPs, d.h. ein Gemisch aus jeweils 1 mM dATP, dGTP, dCTP und dTTP |
| 0,4 | mg/ml BSA (Rinderserumalbumin) |
| 20 | µM Initiatornukleinsäure |
| 20 | nM Templatenukleinsäure |
| 0,25 | mg/ml Polymerase, beispielsweise HIV-I-Reverse Transkriptase (Heterodimer aus p66- und p51-Untereinheit, beide N-terminal mit einem Histidin-Tag versehen; vgl. Le Grice et al. (1995) Methods Enzymol. 262, 130-144) |

Üblicherweise wird das Enzym mit Polymerase-Aktivität in einer Konzentration von 40 mg/ml bis 10⁻⁶ mg/ml eingesetzt. Gemäß einer bevorzugten Ausführungsform wird das Enzym in einer Endkonzentration von 4 mg/ml bis 10⁻⁵ mg/ml eingesetzt, wobei eine Endkonzentration von 0,4 mg/ml bis 0,002 mg/ml besonders bevorzugt ist.

Erfindungsgemäß wird eine beliebige einzelsträngige Nukleinsäure bzw. eine beliebige Nukleinsäure mit einem an ihrem 3'-Ende lokalisierten einzelsträngigen Bereich als Initiator eingesetzt. Generell hat die Initiatornukleinsäure eine Länge von mindestens einem Nukleotid. Vorzugsweise wird eine Initiatornukleinsäure mit einer Länge von mindestens drei Nukleotiden, besonders bevorzugt mit einer Länge von mindestens 15 Nukleotiden, eingesetzt.

Üblicherweise kann die Endkonzentration der Initiatornukleinsäure zwischen 0,2 mM und 2 pM betragen. In einer bevorzugten Ausführungsform wird sie in einer Endkonzentration von 0,2 mM bis 20 nM und besonders bevorzugt in einer Endkonzentration von 20 µM bis 2 µM eingesetzt.

Das mindestens eine lineare Templatemolekül wird erfindungsgemäß in einer Endkonzentration von 0,2 mM bis zum einem einzigen Molekül in einer gegebenen Volumeneinheit eingesetzt. Vorzugsweise beträgt die Endkonzentration des Templates zwischen 5 µM und 20 pM, besonders bevorzugt zwischen 200 nM und 2 nM.

Allgemein eignet sich jedes Enzym mit Polymeraseaktivität für den Einsatz in der IDA-Reaktion. Allerdings können sich aus der Verwendung einer bestimmten Polymerase hinsichtlich der Effizienz, mit der die Reaktion abläuft, der Endkonzentration im jeweiligen Reaktionsansatz, der speziellen Aufgabenstellung in einem bestimmten Anwendungsgebiet oder der anderen in dem Reaktionsansatz vorhandenen Komponenten Unterschiede bzw. verschiedene Anforderungen ergeben.

In einer bevorzugten Ausführungsform handelt es sich bei der Polymerase um eine Reverse Transkriptase oder um eine DNA-abhängige DNA-Polymerase, beispielsweise Klenow-Exo⁻ aus *Escherichia coli* (Derbyshire et al. (1988) Science 240, 199-201), besonders bevorzugt um eine HIV-I-Reverse Transkriptase und am meisten bevorzugt um eine mit einem Histidin-Tag versehene HIV-I-Reverse Transkriptase.

Die IDA-Reaktion wird erfindungsgemäß bei einer Temperatur zwischen 0 °C und 100 °C, eventuell sogar noch darüber, durchgeführt. Bevorzugt wird eine Reaktionstemperatur zwischen 20 °C und 55 °C, besonders bevorzugt eine Reaktionstemperatur zwischen 30 °C und 45 °C. Wird eine Hitze-stabile Polymerase, wie z.B. Taq-DNA-Polymerase, eingesetzt, beträgt die Reaktionstemperatur üblicherweise mindestens 0 °C, bevorzugt mindestens 50 °C.

Die Dauer der Inkubation richtet sich nach den gewünschten Reaktionsprodukten, der speziellen Anwendung der IDA-Reaktion bzw. dem gewünschten Ausmaß der Amplifikation.

Die folgenden Ausführungsbeispiele dienen lediglich der Veranschaulichung der Erfindung.

### Beispiel 1:

Zwei Oligonukleotide mit einer Länge von 20 Nukleotiden wurden in einer Endkonzentration von jeweils 2 µM in 50 µl-Reaktionsansätzen mit verschiedenen Konzentrationen an HIV-I-Reverse Transkriptase für 2 h bei 42 °C inkubiert. Die Reaktionsansätze enthielten:

| | |
|---|---|
| 40 mM | Tris/HCl pH 8,0 |
| 5 mM | KCl |
| 5 mM | Dithiothreitol |
| 2 mM | vSpermidin |
| 15 mM | MgCl₂ |
| 1 mM | dNTPs, d.h. ein Gemisch aus jeweils 1 mM dATP, dGTP, dCTP und dTTP |

Die verwendeten Primer hatten folgende Sequenzen:
5'-CCTCTGCAGACTACTATTAC-3' (P1CATCH, unphosphoryliert) und
5'-CCTGAATTCTTGCTGTGACG-3' (P2CATCH, unphosphoryliert).

Als Polymerase wurde eine mit einem His-tag versehene HIV-I-Reverse Transkriptase eingesetzt, die zuvor mit Hilfe üblicher Proteinaufreinigungsmethoden präpariert worden war (vgl. Protein Purification, Princip. High Res. Meth. and Appl., Janson und Ryden, VCH Publishers, New York, 1989). Diese rekombinate HIV-I-Reverse Transkriptase sowie ihre Aufreinigung und Charakterisierung ist ausführlich beschrieben in Le Grice et al. (1995) Methods Enzymol. 262, 130-144).

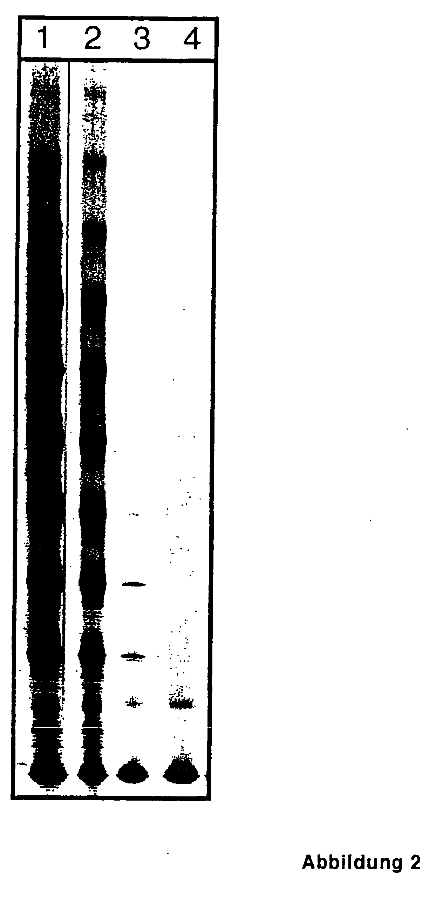

1/25 der Menge eines der beiden Primer wurde als fluoreszenzmarkierte Komponente zugesetzt (Primer P2CATCH, am 5'-Ende mit dem Farbstoff IRD-41 (MWG Biotech, Ebersberg) verknüpft). Zur Analyse der fluoreszenzmarkierten Reaktionsprodukte wurde 1/4 des mittels Mobispin-Säulen (MOBITEC, Göttingen) entsalzten Gesamtreaktionsansatzes auf einem 10%igen, denaturierenden Polyacrylamid-Gel in einem LI-COR-Sequenzautomaten (MWG Biotech, Ebersberg) aufgetrennt. Das Resultat der schrittweisen Verlängerung der doppelsträngigen Reaktionsprodukte um jeweils 20 Basenpaare ist in Abbildung 2 als Molekulargewichtsleiter erkennbar.

Bahn 1 zeigt den Reaktionsansatz mit einer Endkonzentration an HIV-I-Reverse Transkriptase von 0,4 µg/µl, Bahn 2 mit einer Endkonzentration von 0,16 µg/µl, Bahn 3 mit einer Endkonzentration von 0,04 µg/µl und Bahn 4 mit einer Endkonzentration von 0,008 µg/µl.

Für die Sequenzanalyse wurden die Reaktionsprodukte subkloniert und einzelne Klone sequenziert. Diese Analyse zeigte, daß die entstandenen Moleküle ein zentralen Element von 33 Basen Länge enthielten, das durch Selbsthybridisierung des Primers P2CATCH über das 3'-Ende und nachfolgendes Auffüllen zum Doppelstrang entstand, sowie eine Vielzahl miteinander fusionierter Sequenzen der beiden Primer.

Die initiale Doppelstrangbildung war notwendige Voraussetzung für die Oligomerisierung der Primer. Wurde die Reaktion nur mit einem Primer betrieben, dessen Sequenz eine Selbsthybridisierung ausschloß, wurden keine Produkte erhalten. Durch sukzessives Reduzieren des Reaktionsansatzes auf die unbedingt notwendigen Komponenten zeigte sich, daß die Reaktion von der Reversen Transkriptase katalysiert wurde und daß sie nur stattfand, wenn alle vier dNTPs (dATP, dCTP, dGTP und dTTP) anwesend waren.

In nachfolgenden Experimenten konnte bestätigt werden, daß die IDA-Reaktion nicht nur durch eine Reverse Transkriptase, sondern auch durch andere bekannten Polymerasen katalysiert wird.

### Beispiel 2:

Iteratives Kopieren eines doppelsträngigen Templates ausgehend von einem Initiator, der homolog zu einem der beiden 5'-Enden des Templates ist:

Ein doppelsträngiges DNA-Template von 106 Basenpaar Länge (SP6CATCH) wurde in einer Endkonzentration von 20 nM in einem 50 µl-Reaktionsansatz mit HIV-I-Reverse Transkriptase (0,4 µg/µl Endkonzentration) und einem einzelsträngigen Initiator von einer Länge von 20 Basen (P1CATCH, 5 µM Endkonzentration), der zu einem der beiden 5'-Enden des Templates homolog war, für 2 h bei 42 °C inkubiert. Der Reaktionsansatz enthielt weiterhin:

| | |
|---|---|
| 40 | mM Tris pH 7,5 |
| 15 | mM MgCl₂ |
| 16,6 | mM NaCl |
| 1 | mM dNTPs, d.h. ein Gemisch aus jeweils 1 mM dATP, dGTP, dCTP und dTTP |
| 0,4 | mg/ml BSA (Rinderserumalbumin) |

Das verwendete Template und der verwendete Initiator hatten folgende Sequenzen:

| | |
|---|---|
| Initiator: | 5'-CCTCTGCAGACTACTATTAC-3' (P1CATCH, unphosphoryliert, PstI-Erkennungssequenz unterstrichen) |
| Template: | 5'-CCTCTGCAGACTACTATTACATAATACGACTCACTAT AGGGATCTGCACGTATACTTCTATAGTGTCACCTAAATAG GCAGTCTGTCGTCACAGCAAGAATTCAGG-3' |
| | (SP6CATCH, *Pst*I- und *Eco*RI-Erkennungs-sequenzen unterstrichen). |

Im Verlauf der Reaktion vollzog sich, ausgehend von den 3'-Enden des Templates und des Initiators, ein iterativer Kopiervorgang, der mit einer schrittweisen Verlängerung des Templates um mehrere Initiatorkopien und mit einer Netto-Amplifikation verbunden war.

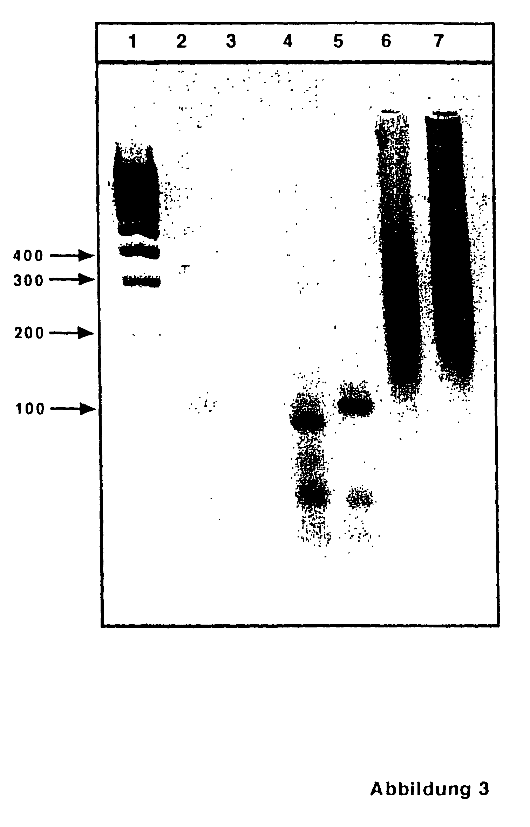

Die Reaktionsprodukte wurden mittels Mobispin-Säulen entsalzt und mittels Gelelektrophorese analysiert (Abbildung 3). Jeweils 1/10 des Gesamtreaktionsansatzes wurde entweder direkt (Bahn 7) oder nach Behandlung mit den Restriktionsendonukleasen *Eco*RI (Bahn 6), *Pst*I (Bahn 5) oder *Pst*I und *Eco*RI (Bahn 4) auf einem 3%igen Agarosegel aufgetrennt. Bahn 1 zeigt 1,5 µg einer 100 Basenpaar-Molekulargewichtsleiter (MBI Fermentas, Litauen). Die Größen der relevanten Banden sind am Rand angegeben. In Bahn 2 und 3 wurde Ausgangstemplate in einer Menge von 1 pmol bzw. 0,2 pmol (entspricht der Menge an Ausgangstemplate in Bahn 4-7) aufgetragen.

Die Reaktion führt zu einer Verteilung der Reaktionsprodukte über einen großen Molekulargewichtsbereich oberhalb der Ausgangstemplatelänge (Bahn 7). Restriktionsverdau mit *Eco*RI, dessen Erkennungssequenz nahe dem einen Ende des Templates lokalisiert ist, beseitigt die Heterogenität der Reaktionsprodukte nicht, verringert jedoch ihre Länge. Schneiden mit PstI, dessen Erkennungssequenz sowohl nahe dem 5'-Ende des Initiators als auch nahe dem anderen Ende des Templates lokalisiert ist, führt zu einer dominanten spezifischen Bande, die ein gegenüber dem Ausgangstemplate um einige Basen verlängertes Produkt repräsentiert. Behandlung mit beiden Restriktionsenzymen führt zu einem gegenüber dem Ausgangstemplate um einige Basen verkürzten Produkt.

Die Daten unterstützen die Annahme, daß im Verlauf der Reaktion mehrere Kopien des Initiators mit beiden Seiten des Templates fusioniert wurden:

Durch *Eco*RI-Behandlung werden nur die auf einer Seite des Templates fusionierten Initiatoren entfernt, so daß die Heterogenität der Produkte bei ihrer gleichzeitigen Verkürzung erhalten bleibt. Restriktionsverdau mit PstI führt auf der PstI-Seite des Templates zur Entfernung von 4 Basen der Templatesequenz sowie aller mit ihr fusionierten Initiatoren. Auf der *Eco*RI-Seite des Templates bleibt zusätzlich zur Templatesequenz eine um 4 Basen verkürzte Initiatorsequenz erhalten. Unter der Annahme, daß die Initiatoren direkt, d.h. ohne Überlappung mit der Templatesequenz fusioniert wurden, wird eine Länge des Produktes von 118 Basen erwartet. Schneiden der Reaktionsprodukte mit *Pst*I und *Eco*RI entfernt je 4 Basen des Templates sowie alle fusionierten Initiatoren auf beiden Seiten des Templates. Das resultierende Produkt hat eine erwartete Länge von 98 Basen.

Durch Klonierung der Reaktionsprodukte und anschließende Sequenzanalyse konnte eine direkte, größtenteils überlappungsfreie Fusionierung von Template und Initiatorsequenz an beiden Enden des Templates bestätigt werden, wobei in einigen Fällen eine Insertion bzw. Deletion weniger Basen detektiert wurde. Eine Netto-Amplifikation des Templates im Verlauf der Reaktion wird bei Vergleich der Bahnen 3 und 4 deutlich.

### Beispiel 3:

Iteratives Kopieren eines doppelsträngigen Templates ausgehend von einem zum Template nichthomologen Initiator:

Ein doppelsträngiges DNA-Template mit einer Länge von 106 Basenpaaren (SP6CATCH) wurde in einer Endkonzentration von 20 nM in einem 50 µl-Reaktionsansatz mit HIV-1-Reverse Transkriptase (0,4 µg/µl Endkonzentration) und einem einzelsträngigen Initiator von 35 Basen Länge (LOOP, 20 µM Endkonzentration), der zu keinem der beiden 5'-Enden des Templates homolog war, für 2 h bei 42 °C inkubiert. Der Reaktionsansatz enthielt weiterhin:

| | |
|---|---|
| 40 | mM Tris pH 7,5 |
| 15 | mM MgCl₂ |
| 16,6 | mM NaCl |
| 1 | mM dNTPs, d.h. ein Gemisch aus jeweils 1 mM dATP, dGTP, dCTP und dTTP |
| 0,4 | mg/ml BSA (Rinderserumalbumin) |

Das verwendete Template und der verwendete Initiator hatten folgende Sequenzen:

| | |
|---|---|
| Initiator: | 5'-GTTGATTTATTTAATTCATAAATAAAAATCCCT-3' (LOOP, unphosporyliert) |
| Template: | 5'-CCTCTGCAGACTACTATTACATAATACGACTCACTA TAGGGATCTGCACGTATACTTCTATAGTGTCACCTAAAT AGGCAGTCTGTCGTCACAGCAAGAATTCAGG-3' |
| | (SP6CATCH, *Pst*I- und *Eco*RI-Erkennungs-sequenzen unterstrichen). |

Im Verlauf der Reaktion vollzog sich, ausgehend von den 3'-Enden des Templates und des Initiators ein iterativer Kopiervorgang, der mit einer schrittweisen

Verlängerung des Templates um mehrere Initiatorkopien und mit einer Netto-Amplifikation verbunden war.

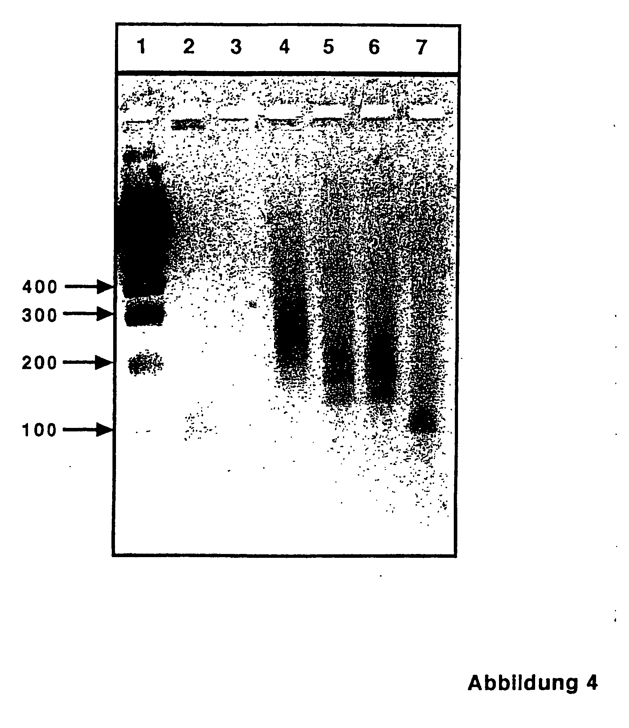

Die Reaktionsprodukte wurden mittels Mobispin-Säulen entsalzt und mittels Gelelektrophorese analysiert (Abbildung 4). Jeweils 1/10 des Gesamtreaktionsansatzes wurde entweder direkt (Bahn 4) oder nach Behandlung mit den Restriktionsendonukleasen *Eco*RI (Bahn 6), *Pst*I (Bahn 5) oder *Pst*I und *Eco*RI (Bahn 7) auf einem 3%igen Ethidiumbromid-gefärbten Agarosegel aufgetrennt. Bahn 1 zeigt 1,5 µg einer 100 Basenpaar-Molekulargewichtsleiter (MBI Fermentas, Litauen). Die Größen der relevanten Banden sind am Rand angegeben. In Bahn 2 und 3 wurde Ausgangstemplate in einer Menge von 2 pmol bzw. 0,2 pmol (entspricht der Menge an Ausgangstemplate in Bahn 4-7) aufgetragen.

Die Reaktion führt zu einer Verteilung der Reaktionsprodukte über einen großen Molekulargewichtsbereich oberhalb der Ausgangstemplatelänge (Bahn 4). Die Behandlung mit *Eco*RI bzw. *Pst*I allein, deren Erkennungssequenzen jeweils an einem der beiden Enden des Templates lokalisiert sind, beseitigt die Heterogenität der Reaktionsprodukte nicht, verringert jedoch ihre Länge (Bahn 6 bzw. 5). Schneiden mit beiden Restriktionsenzymen führt zu einem gegenüber dem Ausgangstemplate um einige Basen verkürzten Produkt (Bahn 7).

Die Daten unterstützen die Annahme, daß im Verlauf der Reaktion mehrere Kopien des Initiators mit beiden Seiten des Templates fusioniert wurden:

Durch *Eco*RI bzw. *Pst*I-Behandlung allein werden nur die auf einer Seite des Templates fusionierten Initiatoren entfernt, so daß die Heterogenität der Produkte bei ihrer gleichzeitigen Verkürzung erhalten bleibt. Ein Restriktionsverdau der Reaktionsprodukte mit *Pst*I und *Eco*RI entfernt je 4 Basen des Templates sowie alle fusionierten Initiatoren auf beiden Seiten des Templates. Das resultierende Produkt hat eine erwartete Länge von 98 Basen.

Klonierung der Reaktionsprodukte und anschließende Sequenzanalyse bestätigten eine größtenteils überlappungsfreie Fusionierung von Template und Initiatorsequenz an beiden Enden des Templates, wobei in einigen Fällen eine Insertion bzw. Deletion weniger Basen detektiert wurde. Besonders häufig wurden nach der Klonierung Mutationen innerhalb der Initiatorsequenz detektiert. Eine Netto-Amplifikation des Templates im Verlauf der Reaktion wird bei Vergleich der Bahnen 3 und 7 deutlich.

### Beispiel 4:

Kombination der IDA-Reaktion mit einer konventionellen Polymerase-Ketten-Reaktion (PCR) schafft die Möglichkeit, ein Template in der PCR mit Primern zu amplifizierten, die keine Homologie zum Template aufweisen.

Ein doppelsträngiges DNA-Template von 106 Basen Länge (SP6CATCH) wurde in einer Endkonzentration von 10 nM in einem 50 µl-Reaktionsansatz mit unterschiedlichen Mengen an HIV-I-Reverse Transkriptase und einem einzelsträngigen Initiator von 35 Basen Länge (LOOP, 10 µM Endkonzentration), der zu keinem der beiden 5'-Enden des Templates homolog war, für 10 min. bei 42 °C inkubiert. Der Reaktionsansatz enthielt weiterhin:

| | |
|---|---|
| 1 x | Taq-Puffer (Promega, Madison, WI, USA) |
| 2 mM | MgCl₂ |
| 200 µM | dNTPs, d.h. ein Gemisch aus jeweils 200 µM dATP, dGTP, dCTP und dTTP |

Danach wurden 2,5 Einheiten Taq-Polymerase (Promega) hinzugegeben und der Reaktionsansatz mit 40 µl Mineralöl überschichtet. Der Ansatz wurde im Thermocycler über 20 Zyklen unter folgendem Temperaturregime inkubiert:

| | |
|---|---|
| 1. Zyklus: | 5 min. 95 °C, 1 min. 45 °C, 1 min. 72 °C und |
| 2.-20. Zyklus: | 1 min. 95 °C, 1 min. 45 °C, 1 min. 72 °C. |

Das verwendete Template und der verwendete Initiator hatten folgende Sequenzen:

| | |
|---|---|
| Initiator: | 5'-GTTGATATTTATTTAATTCATAAATAAAAATCCCT-3' (LOOP, unphosporyliert) |
| Template: | 5'-CCTCTGCAGACTACTATTACATAATACGACTCACTAT AGGGATCTGCACGTATACTTCTATAGTGTCACCTAAATAG GCAGTCTGTCGTCACAGCAAGAATTCAGG-3' |
| | (SP6CATCH, *Pst*I- und *Eco*RI-Erkennungs-sequenzen unterstrichen). |

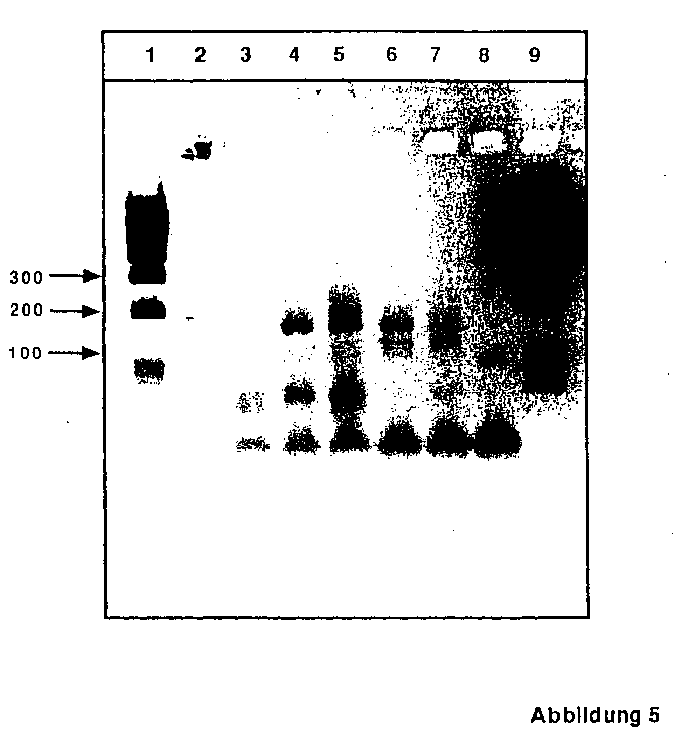

Nach Ablauf der Reaktion wurde 1/10 des Reaktionsansatzes zur Analyse auf einem 3%igen Agarose-Gel aufgetrennt (Abbildung 5).

Abbildung 5 zeigt die Reaktionsprodukte von Parallelexperimenten, die sich lediglich in der Menge der zugesetzten HIV-I-Reverse Transkriptase unterschieden. Diese betrug 0,4 µg/µl Endkonzentration (Bahn 2), 0,12 µg/µl Endkonzentration (Bahn 3), 0,04 µg/µl Endkonzentration (Bahn 4), 0,012 µg/µl Endkonzentration (Bahn 5), 0,004 µg/µl Endkonzentration (Bahn 6), 0,0012 µg/µl Endkonzentration (Bahn 7) und keine Zugabe von HIV-I-Reverse Transkriptase (Bahn 8).

Nach Vorinkubation mit HIV-I-Reverse Transkriptase wird im Verlauf der PCR ein Fragment amplifiziert, dessen Länge der Summe aus Templatelänge und doppelter Länge des Initiators entspricht (ca. 176 Basen). Die Amplifikation dieses Produktes ist besonders effizient bei Vorinkubation mit mittleren Konzentration an HIV-I-Reverse Transkriptase (Bahnen 4 bis 6). Vorinkubation des Templates ohne HIV-I-Reverse Transkriptase läßt das Template unverändert und führt im Verlauf der PCR nicht zu dessen Amplifikation (Bahn 8).

Die Reaktionsprodukte wurden durch Behandlung mit Restriktionsendonukleasen charakterisiert. Schneiden des Reaktionsproduktes mit *Eco*RI bzw. *Pst*I allein, deren Erkennungssequenzen jeweils an einem der beiden Enden lokalisiert sind, führt zur Entfernung eines der beiden Initiatoren und 4 zusätzlichen Basen des Templates und resultiert in einer Verkürzung des Reaktionsproduktes um ca. 39 Basen. Schneiden mit beiden Enzymen führt zum Entfernen beider Initiatoren und 8 zusätzlichen Basen des Templates und somit zu einer Verkürzung des Reaktionsproduktes um ca. 78 Basen.

Zur weiteren Analyse wurden die Reaktionsprodukte kloniert und sequenziert. Die Sequenzanalyse bestätigte, daß im Verlauf der Reaktion eine direkte, größtenteils überlappungsfreie Fusionierung von Template und Initiatorsequenz an beiden Enden des Templates erfolgte, wobei in einigen Fällen eine Insertion bzw. Deletion weniger Basen detektiert wurde.

### Beispiel 5:

In Analogie zu Beispiel 1 wurde unter den gleichen Reaktionsbedingungen eine Reaktion durchgeführt, bei der anstelle der HIV-I-Reverse Transkriptase 0,25 Einheiten/µl einer Exonuklease-defizienten Variante des großen Fragmentes der DNA-Polymerase I von *E. coli* (Klenow Exo⁻; Derbyshire et al. (1988) Science 240, 199-201; New England Biolabs, Schwalbach) eingesetzt wurde. Als Ergebnis ergab sich eine vergleichbare Primerleiter, die auf den der IDA-Reaktion innewohnenden Reaktionsmechanismus schließen läßt.

Im Zusammenhang mit den eingesetzten Nukleinsäuremolekülen wurden übliche molekularbiologische Standardmethoden (Sequenzierung, Restriktionsverdaue, Gelelektrophorese, etc.) eingesetzt, wie sie beispielsweise in Sambrook et al (1989) Molecular Cloning: A Laboratory Manual, 2. Auflage, Cold Spring Harbor Laboratory Press, Cold Spring Harbor, New York, beschrieben sind. Im Zusammenhang mit proteinchemischen Methoden, beispielsweise im Verbindung mit der Aufreinigung eingesetzter Enzyme, sei auf das Manual Protein Purification, Princip. High Res. Meth. and Appl., Janson und Ryden, VCH Publishers, New York, 1989 verwiesen. Der Einsatz von Restriktionsenzymen (MBI Fermentas, Litauen), Taq-Polymerase (Promega) u.ä. erfolgte nach Herstellerangaben.

## Patentansprüche

1. Verfahren zur Synthese von Nukleinsäuren,
**gekennzeichnet durch** eine DNA-Polymerase-katalysierte Reaktion, die von einer Hybridisisierung unabhängig **durch** mindestens eine zumindest am 3'-Ende einzelsträngige Nukleinsäure initiiert wird, wobei das Reaktionsprodukt die Sequenz des Initiators enthält, die direkt mit dem 5'-Ende des Templates fusioniert ist.

2. Verfahren nach Anspruch 1, bei dem die zumindest am 3'-Ende einzelsträngige Initiatornukleinsäure in Abhängigkeit von einer Templatesequenz verlängert wird.

3. Verfahren nach Anspruch 1 oder 2, bei dem mindestens ein Strang einer zumindest partiell doppelsträngigen Templatenukleinsäure ausgehend vom freien 3'-OH der Initiatornukleinsäure kopiert wird.

4. Verfahren nach Anspruch 1, bei dem mindestens ein Strang einer zumindest partiell doppelsträngigen Templatenukleinsäure in Abhängigkeit von der Initiatornukleinsäure verlängert wird.

5. Verfahren nach Anspruch 1 oder 4, bei dem mindestens eine zumindest am 3'-Ende einen einzelsträngigen Bereich aufweisende Initiatornukleinsäure ausgehend vom freien 3'-OH eines zumindest partiell doppelsträngigen Templatemoleküls kopiert wird.

6. Verfahren nach einem der vorangehenden Ansprüche, dessen Reaktionsprodukte Nukleinsäuremoleküle umfassen, deren Sequenz einer um mindestens eine Initiatornukleinsäuresequenz verlängerten Templatenukleinsäure entspricht.

7. Verfahren nach einem der vorangehenden Ansprüche, bei dem die Initiatornukleinsäure in einer Endkonzentration von 0,2 mM bis 2 pM vorliegt.

8. Verfahren nach Anspruch 7, bei dem die Initiatornukleinsäure in einer Endkonzentration von 0,2 mM bis 20 nM vorliegt.

9. Verfahren nach einem der vorangehenden Ansprüche, bei dem die zu kopierende Templatenukleinsäure in einer Endkonzentration von 0,2 mM bis zu einem einzigen Molekül vorliegt.

10. Verfahren nach Anspruch 9, bei dem die zu kopierende Templatenukleinsäure in einer Endkonzentration von 5 µM bis 20 pM vorliegt.

11. Verfahren nach einem der vorangehenden Ansprüche, bei dem mindestens ein Enzym mit Polymerase-Aktivität in einer Endkonzentration von 40 mg/ml bis 10⁻⁶ mg/ml vorliegt.

12. Verfahren nach Anspruch 11, bei dem das Enzym mit Polymerase-Aktivität in einer Endkonzentration von 4 mg/ml bis 10⁻⁵ mg/ml vorliegt.

13. Verfahren nach einem der vorangehenden Ansprüche, bei dem das Enzym mit Polymerase-Aktivität eine Reverse Transkriptase ist.

14. Verfahren nach Anspruch 13, bei dem die Reverse Transkriptase eine HIV-I-Reverse Transkriptase ist.

15. Verfahren nach Anspruch 14, bei dem die HIV-I-Reverse Transkriptase mit einem Histidin-Tag versehen ist.

16. Verfahren nach einem der Ansprüche 1 bis 12, bei dem das Enzym mit Polymerase-Aktivität eine DNA-abhängige DNA-Polymerase ist.

17. Verfahren nach Anspruch 16, bei dem die DNA-Polymerase Klenow Exo⁻ aus *Escherichia coli* ist.

18. Verfahren nach einem der vorangehenden Ansprüche, bei dem die Polymerase-katalysierte Reaktion isothermal ist.

19. Verfahren nach einem der vorangehenden Ansprüche, bei dem mindestens eine Initiatornukleinsäure eine Ribonukleinsäure ist.

20. Verfahren nach Anspruch 19, bei dem der RNA-Initiator nach Auffüllen zum Doppelstrang zumindest partiell durch eine Ribonuklease abgebaut wird.

21. Verfahren nach einem der vorangehenden Ansprüche, bei dem mindestens eine Initiatornukleinsäure ein Nukleinsäureanalogon ist.

22. Verfahren nach Anspruch 21, bei dem das Nukleinsäureanalogon Peptide Nucleic Acid (PNA) ist.

23. Verfahren nach einem der vorangehenden Ansprüche, bei dem ein Gemisch von Templatenukleinsäuren kopiert und gegebenenfalls amplifiziert wird.

24. Verfahren nach einem der vorangehenden Ansprüche, bei dem ein Gemisch von Initiatornukleinsäuren eingesetzt wird.

25. Verfahren zur Amplifikation von Nukleinsäuren, bei dem das Verfahren nach einem der vorangehenden Ansprüche einer PCR-Reaktion oder einer anderen primerabhängigen Amplifikationsreaktion vorgeschaltet ist.

26. Verfahren nach Anspruch 1, bei dem Nukleinsäuremoleküle ohne Sequenzkomplementarität miteinander fusioniert werden.

27. Verfahren nach Anspruch 1, bei dem eine einzelsträngige Nukleinsäure als Ausgangstemplate dient und dem hybridisierungsunabhängigen Reaktionsschritt die Hybridisierung einer zum 3'-Ende des Templates komplementären Initiatornukleinsäure an das Template und die Polymerase-katalysierte Auffüllung des Doppelstranges vorangehen.

28. Verfahren zur Umschreibung von RNA in doppelsträngige DNA bei gleichzeitiger Amplifikation, umfassend das Verfahren nach Anspruch 27.

29. Verfahren zur sequenzunspezifischen Fusionierung von Nukleinsäuren, umfassend das Verfahren nach Anspruch 1.

30. Verfahren zum shuffling von DNA ohne Sequenzhomologie, umfassend das Verfahren nach Anspruch 1.

31. Verfahren zur Herstellung von Random-DNA großer Länge, umfassend das Verfahren nach Anspruch 1.

32. Verfahren zur enzymatischen Synthese von Oligonukleotiden beliebiger Sequenz, umfassend das Verfahren nach Anspruch 1.

## Claims

1. Method for synthesis of nucleic acids, **characterized by** a DNA polymerase catalyzed reaction which is initiated independently of a hybridization by at least one nucleic acid which is single-stranded at least at the 3' end, the reaction product comprising the sequence of the initiator which is directly fused to the 5' end of the template.

2. Method according to claim 1, wherein the at least at the 3' end single-stranded initiator nucleic acid is extended depending on a template sequence.

3. Method according to claim 1 or 2, wherein at least one strand of an at least partially double-stranded template nucleic acid is copied starting from the free 3'-OH of the initiator nucleic acid.

4. Method according to claim 1, wherein at least one strand of an at least partially double-stranded template nucleic acid is extended depending on an initiator nucleic acid.

5. Method according to claim 1 or 4, wherein at least one initiator nucleic acid having a single-stranded region at least at the 3' end is copied starting from the free 3'-OH of an at least partially double-stranded template molecule.

6. Method according to any of the preceding claims whose reactions products comprise nucleic acid molecules whose sequence corresponds to a template nucleic acid extended by at least one initiator nucleic acid sequence.

7. Method according to any of the preceding claims, wherein the initiator nucleic acid is present in a final concentration of 0.2 mM to 2 pM.

8. Method according to claim 7, wherein the initiator nucleic acid is present in a final concentration of 0.2 mM to 20 nM.

9. Method according to any of the preceding claims, wherein the template nucleic acid to be copied is present in a final concentration of 0.2 mM up to a single molecule.

10. Method according to claim 9, wherein the template nucleic acid to be copied is present in a final concentration of 5 µM to 20 pM.

11. Method according to any of the preceding claims, wherein at least one enzyme having polymerase activity is present in a final concentration of 40 mg/mL to 10⁻⁶ mg/mL.

12. Method according to claim 11, wherein the enzyme having polymerase activity is present in a final concentration of 4 mg/mL to 10⁻⁵ mg/mL.

13. Method according to any of the preceding claims, wherein the enzyme having polymerase activity is a reverse transcriptase.

14. Method according to claim 13, wherein the reverse transcriptase is an HIV-I reverse transcriptase.

15. Method according to claim 14, wherein the HIV-I reverse transcriptase is provided with a histidine tag.

16. Method according to any of claims 1 to 12, wherein the enzyme with polymerase activity is a DNA-dependent DNA polymerase.

17. Method according to claim 16, wherein the DNA polymerase is Klenow exo⁻ from *Escherichia coli*.

18. Method according to any of the preceding claims, wherein the polymerase catalyzed reaction is isothermal.

19. Method according to any of the preceding claims, wherein at least one initiator nucleic acid is a ribonucleic acid.

20. Method according to claim 19, wherein the RNA initiator is degraded at least partially by a ribonuclease after being filled in to a double strand.

21. Method according to any of the preceding claims, wherein at least one initiator nucleic acid is a nucleic acid analog.

22. Method according to claim 21, wherein the nucleic acid analog is peptide nucleic acid (PNA).

23. Method according to any of the preceding claims, wherein a mixture of template nucleic acids is copied and optionally amplified.

24. Method according to any of the preceding claims, wherein a mixture of initiator nucleic acids is used.

25. Method for amplification of nucleic acids, wherein the method according to any of the preceding claims takes place prior to a PCR reaction or another primer-dependent amplification reaction.

26. Method according to claim 1, wherein the nucleic acid molecules are fused together without sequence complementarity.

27. Method according to claim 1, wherein a single-stranded nucleic acid functions as starting template and the hybridization of an initiator nucleic acid complementary to the 3' end of the template to the template and the polymerase-catalyzed filling in of the double strand take place prior to the hybridization-independent reaction step.

28. Method for converting RNA into double-stranded DNA with simultaneous amplification comprising the method according to claim 27.

29. Method for sequence-nonspecific fusing of nucleic acids, comprising the method according to claim 1.

30. Method for shuffling DNA without sequence homology, comprising the method according to claim 1.

31. Method for producing random long-length DNA, comprising the method according to claim 1.

32. Method for enzymatic synthesis of oligonucleotides of any sequence, comprising the method according to claim 1.

## Revendications

1. Procédé pour la synthèse des acides nucléiques, **caractérisé par** une réaction catalysée par l'ADN polymérase, qui est initiée, indépendamment d'une hybridisation, par au moins un acide nucléique simple brin à au moins l'extrémité 3', dans lequel le produit de réaction contient la séquence de l'initiateur, qui est fusionnée directement à l'extrémité 5' du template.

2. Procédé selon la revendication 1, dans lequel l'acide nucléique initiateur simple brin à au moins l'extrémité 3' est allongé suivant une séquence template.

3. Procédé selon la revendication 1 ou 2, dans lequel au moins un brin d'un acide nucléique template au moins partiellement double brin est copié à partir du 3'-OH libre de l'acide nucléique initiateur.

4. Procédé selon la revendication 1, dans lequel au moins un brin d'un acide nucléique template au moins partiellement double brin est allongé suivant l'acide nucléique initiateur.

5. Procédé selon la revendication 1 ou 4, dans lequel au moins un acide nucléique initiateur ayant une région simple brin à au moins l'extrémité 3' est copié à partir du 3'-OH libre d'une molécule de template au moins partiellement double brin.

6. Procédé selon l'une des revendications précédentes, dont les produits de réaction comprennent des molécules d'acide nucléique, dont la séquence corresponde à un acide nucléique template allongé par au moins une séquence de l'acide nucléique initiateur.

7. Procédé selon l'une des revendications précédentes, dans lequel l'acide nucléique initiateur est présent à une concentration finale de 0,2 mM à 2 pM.

8. Procédé selon la revendication 7, dans lequel l'acide nucléique initiateur est presente à une concentration finale de 0,2 mM à 20 nM.

9. Procédé selon l'une des revendications précédentes, dans lequel l'acide nucléique template qui est à copier est presente à une concentration finale de 0,2 mM jusqu'une seule molécule.

10. Procédé selon la revendication 9, dans lequel l'acide nucléique template à copier est presente à une concentration finale de 5 µM à 20 pM.

11. Procédé selon l'une des revendications précédentes, dans lequel au moins une enzyme ayant l'activité polymerase est présente à une concentration finale de 40 mg/ml à 10⁻⁶ mg/ml.

12. Procédé selon la revendication 11, dans lequel l'enzyme ayant l'activité polymerase est présente à une concentration finale de 4 mg/ml à 10⁻⁵ mg/ml.

13. Procédé selon l'une des revendications précédentes, dans lequel l'enzyme ayant l'activité polymérase est une transcriptase inverse.

14. Procédé selon la revendication 13, dans lequel la transcriptase inverse est une transcriptase inverse HIV-1.

15. Procédé selon la revendication 14, dans lequel la transcriptase inverse HIV-I est munie d'une étiquette histidine.

16. Procédé selon l'une des revendications 1 à 12, dans lequel l'enzyme ayant l'activité polymérase est une ADN polymérase dépendante de l'ADN.

17. Procédé selon la revendication 16, dans lequel l'ADN polymérase est Klenow Exo d'*Escherichia coli.*

18. Procédé selon l'une des revendications précédentes, dans lequel la réaction catalysée par la polymérase est isothermale.

19. Procédé selon l'une des revendications précédentes, dans lequel au moins un acide nucléique initiateur est un acide ribonucléique.

20. Procédé selon la revendication 19, dans lequel l'initiateur de l'ARN est décomposé au moins partiellement par une ribonucléase après le remplissage jusqu'au brin double.

21. Procédé selon l'une des revendications précédentes, dans lequel au moins un acide nucléique initiateur est un analogue d'acide nucléique.

22. Procédé selon la revendication 21, dans lequel l'analogue d'acide nucléique est l'Acide Nucléique Peptidique (ANP).

23. Procédé selon l'une des revendications précédentes, dans lequel un mélange d'acides nucléiques templates est copié et en cas échéant est amplifié.

24. Procédé selon l'une des revendications précédentes, dans lequel un melange d'acides nucléiques initiateurs est mis en oeuvre.

25. Procédé pour l'amplification des acides nucléiques, dans lequel le procédé selon l'une des revendications précédentes est en amont d'une réaction PCR ou d'une autre réaction d'amplification dépendante d'amorce.

26. Procédé selon la revendication 1, dans lequel des molécules d'acide nucléique dépourvues de complémentarité de séquence sont fusionnées l'un avec l'autre.

27. Procédé selon la revendication 1, dans lequel un acide nucléique simple brin sert comme des templates de départ, et l'hybridisation au template d'un acide nucléique initiateur complémentaire à l'extrémité 3' du template et le remplissage du double brin, qui est catalysé par la polymerase, sont en aval de l'étape de réaction indépendante de l'hybridisation.

28. Procédé pour la transcription de l'ARN en l'ADN double brin pendant l'amplification simultané, comprenant le procédé selon la revendication 27.

29. Procédé pour la fusion non spécifique de séquence des acides nucléiques comprenant le procédé selon la revendication 1.

30. Procédé pour le réarrangement de l'ADN dépourvu de complémentarité de séquence comprenant le procédé selon la revendication 1.

31. Procédé pour la fabrication de l'ADN aléatoire de la longueur grande comprenant le procédé selon la revendication 1.

32. Procédé pour la synthèse enzymatique des oligonucleotides de séquence quelconque comprenant le procédé selon la revendication 1.
